# EUROPÄISCHE PATENTSCHRIFT

(11) **EP 1 375 560 B1**
(45) Veröffentlichungstag und Bekanntmachung des Hinweises auf die Patenterteilung: **22.03.2006**
(21) Anmeldenummer: 03013779.8
(22) Anmeldetag: 18.06.2003
(51) Int. Cl.: C08G 73/00, C08G 61/12

(54) **Alkylendioxythiophen-Dimere und Trimere**
Alkylenedioxythiophene dimers and trimers
Dimères et trimères de alkylènedioxythiophène

(30) Priorität: 28.06.2002 DE 10229218
(43) Veröffentlichungstag der Anmeldung: 02.01.2004
(73) Patentinhaber: H.C. Starck GmbH, 38642 Goslar (DE)
(72) Erfinder: Reuter, Knud, Dr., 47800 Krefeld (DE); Nikanorov, Valery A., Dr., 105062 Moskau (RU); Bazhenov, Vassily, M., Dr., Moskau 127566 (RU)
(74) Vertreter: Zobel, Manfred

(56) Entgegenhaltungen:
- US-A1- 2001 034 453
- US-A1- 2002 077 450
- GROENENDAAL L ET AL: "POLY(3,4-ETHYLENEDIOXYTHIOPHENE) AND ITS DERIVATIVES: PAST, PRESENT, AND FUTURE" ADVANCED MATERIALS, VCH VERLAGSGESELLSCHAFT, WEINHEIM, DE, Bd. 12, Nr. 7, 4. April 2000 (2000-04-04), Seiten 481-494, XP000949752 ISSN: 0935-9648

## Beschreibung

Die Erfindung betrifft neue 3,4-Alkylendioxythiophen-Dimere und -Trimere, ihre Herstellung und ihre Verwendung als Vorprodukte für leitfähige Polymere.

Die Verbindungsklasse der *π*-konjugierten Polymere war in den letzten Jahrzehnten Gegenstand zahlreicher Veröffentlichungen. Sie werden auch als leitfähige Polymere oder als synthetische Metalle bezeichnet.

Wegen der erheblichen Delokalisierung der *π*-Elektronen entlang der Hauptkette zeigen diese Polymere interessante (nichtlineare) optische Eigenschaften, und nach Oxidation oder Reduktion stellen sie gute elektrische Leiter dar. Dadurch werden diese Verbindungen voraussichtlich eine führende und aktive Rolle auf verschiedenen praktischen Anwendungsgebieten übernehmen, wie z.B. in der Datenspeicherung, der optischen Signalverarbeitung, der Unterdrückung elektromagnetischer Störungen (EMI) und der Sonnenenergieumwandlung, sowie in wiederaufladbaren Batterien, lichtemittierenden Dioden, Feldeffekttransistoren, Leiterplatten, Sensoren und antistatischen Materialien.

Beispiele für bekannte *π*-konjugierte Polymere sind Polypprole, Polythiophene, Polyaniline, Polyacetylene, Polyphenylene und Poly(p-phenylen-vinylene).

Ein besonders wichtiges und technisch genutztes Polythiophen ist das Poly(ethylen-3,4-dioxythiophen), das in seiner oxidierten Form sehr hohe Leitfähigkeiten aufweist und beispielsweise in der EP 339 340 A2 beschrieben ist. Eine Übersicht über zahlreiche Poly(alkylen-3,4-dioxythiophen)-Derivate, insbesondere Poly(ethylen-3,4-dioxythiophen)-Derivate, deren Monomerbausteine, Synthesen und Anwendungen gibt L. Groenendaal, F. Jonas, D. Freitag, H. Pielartzik & J. R. Reynolds, Adv. Mater. **12**, (2000) S. 481 - 494.

Sehr hohe Leitfähigkeiten werden auch durch die Methodik der *in situ*-Polymerisation erzielt, bei der das monomere Ethylen-3,4-dioxythiophen mit Oxidationsmitteln, wie z.B. Eisen-III-tosylat, in Lösung zu einer hochleitfähigen Schicht aus oxidiertem Poly(ethylen-3,4-dioxythiophen) umgesetzt wird. Diese Vorgehensweise wird z.B. für die Herstellung von Kondensatoren genutzt.

Obwohl somit bereits verschiedene gut geeignete Techniken zur Herstellung von leitfähigen Poly(ethylen-3,4-dioxythiophen)en existieren, besteht Bedarf an weiteren Verbesserungen. Insbesondere werden Wege und Zwischenprodukte gesucht, die eine sehr rasche Bildung leitfähiger Poly(ethylen-3,4-dioxythiophen)e ermöglichen.

Diese Aufgabe wurde durch die Bereitstellung neuer, gegebenenfalls substituierter 3,4-Alkylendioxythiophen-Dimere und -Trimere gelöst.

Gegenstand der vorliegenden Erfindung sind somit Verbindungen der allgemeinen Formel I, 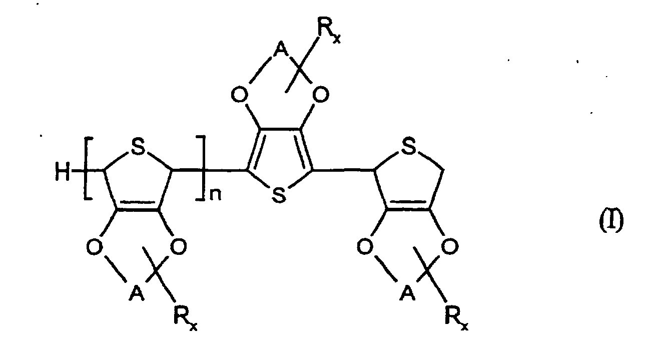 worin
- A: für einen gegebenenfalls substituierten C₁-C₄-Alkylenrest, bevorzugt für einen gegebenenfalls substituierten C₂-C₃-Alkylenrest, steht,
- R: für einen oder mehrere, gleiche oder verschiedene lineare(n) oder verzweigte(n), gegebenenfalls substituierte(n) C₁-C₁₈-Alkylrest(e), bevorzugt lineare(n) oder verzweigte(n), gegebenenfalls substituierte(n) C₁-C₁₄-Alkylrest(e), gegebenenfalls substituierte(n) C₅-C₁₂-Cycloalkylrest(e), gegebenenfalls substituierte(n) C₆-C₁₄-Arylrest(e), gegebenenfalls substituierte(n) C₁-C₄-Hydroxyalkylrest(e), bevorzugt gegebenenfalls substituierte(n) C₁-C₂₋Hydroxyalkylrest(e), oder einen oder mehrere Hydroxylrest(e) steht,
- x: für eine ganze Zahl von 0 bis 8 steht, bevorzugt von 0 bis 6, besonders bevorzugt für 0 oder 1 steht, und
- n: für 0 oder 1 steht.

Formel I ist im Sinne der Erfindung so zu verstehen, dass der Rest A gegebenenfalls an x beliebigen Stellen durch x Reste R substituiert ist, wobei auch jeweils zwei Reste R am gleichen Kohlenstoffatom von A gebunden sein können.

Ebenfalls Gegenstand der Erfindung sind Gemische aus Verbindungen der allgemeinen Formel I, wobei diese Mischungen aus Verbindungen mit unterschiedlichen Resten R, mit unterschiedlicher Anzahl x der Reste R sowie mit unterschiedlichem n sein können.

Bevorzugt Gegenstand der Erfindung sind Verbindungen der allgemeinen Formeln I-a oder I-b, 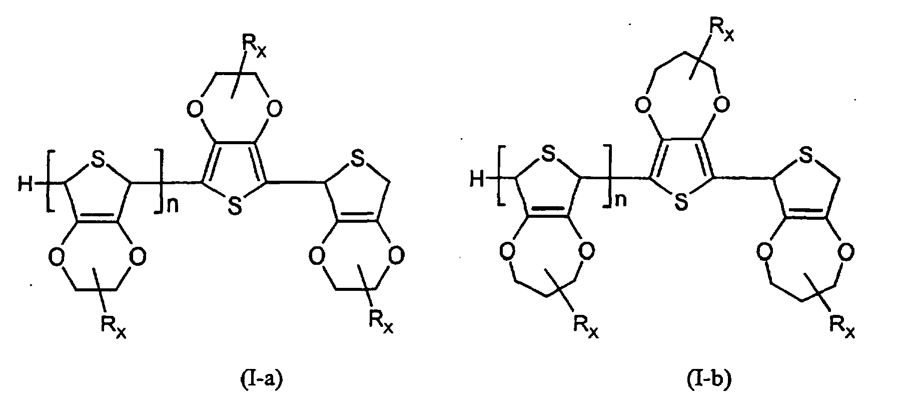 worin
- R: für einen oder mehrere, gleiche oder verschiedene lineare(n) oder verzweigte(n), gegebenenfalls substituierte(n) C₁-C₁₈-Alkylrest(e), bevorzugt lineare(n) oder verzweigte(n), gegebenenfalls substituierte(n) C₁-C₁₄-Alkylrest(e), gegebenenfalls substituierte(n) C₅-C₁₂-Cycloalkylrest(e), gegebenenfalls substituierte(n) C₆-C₁₄-Arylrest(e), gegebenenfalls substituierte(n) C₁-C₄-Hydroxyalkylrest(e), bevorzugt gegebenenfalls substituierte(n) C₁-C₂₋Hydroxyalkylrest(e), oder einen oder mehrere Hydroxylrest(e) steht,
- x: für eine ganze Zahl von 0 bis 8 steht, bevorzugt von 0 bis 6, besonders bevorzugt für 0 oder 1 steht, und
- n: für 0 oder 1 steht.

Besonders bevorzugt sind dies Verbindungen der allgemeinen Formeln I-a-1 oder I-b-1, 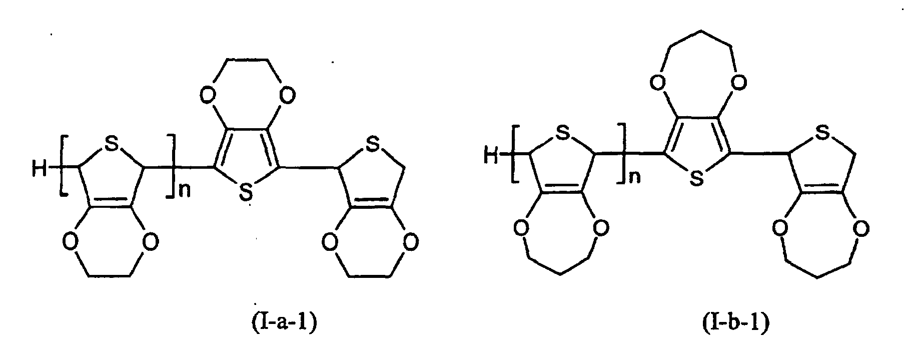 worin
- n: für 0 oder 1 steht.

In einer bevorzugten Ausführungsform sind dies dimere Verbindungen der Formeln I-a-1a oder I-a-2a, 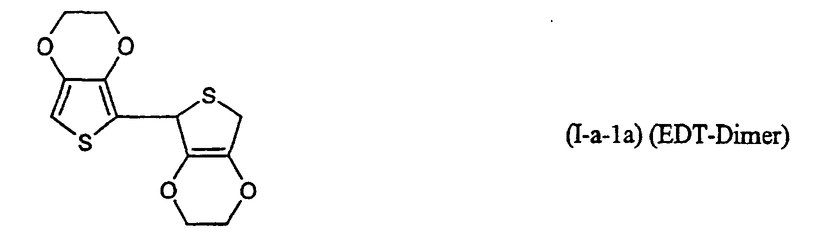 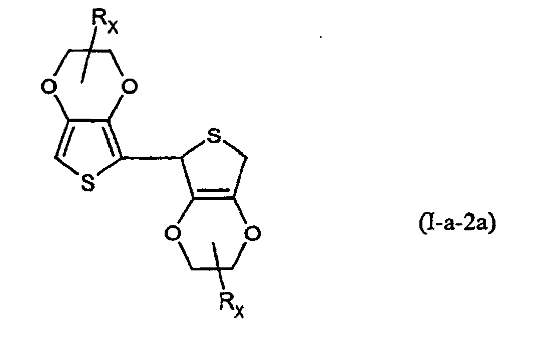 worin
- R: für einen oder mehrere, gleiche oder verschiedene lineare(n) oder verzweigte(n), gegebenenfalls substituierte(n) C₁-C₁₈-Alkylrest(e), bevorzugt lineare(n) oder verzweigte(n), gegebenenfalls substituierte(n) C₁-C₁₄-Alkylrest(e), gegebenenfalls substituierte(n) C₅-C₁₂-Cycloalkylrest(e), gegebenenfalls substituierte(n) C₆-C₁₄-Arylrest(e), gegebenenfalls substituierte(n) C₁-C₄-Hydroxyalkylrest(e), bevorzugt gegebenenfalls substituierte(n) C₁-C₂₋Hydroxyalkylrest(e), oder einen oder mehrere Hydroxylrest(e) steht, und
- x: für eine ganze Zahl von 0 bis 8 steht, bevorzugt von 0 bis 6, besonders bevorzugt für 0 oder 1 steht,
gegebenenfalls im Gemisch mit monomeren Edukten.

In einer weiteren bevorzugten Ausführungsform sind dies trimere Verbindungen der Formeln I-a-1b und I-a-2b, 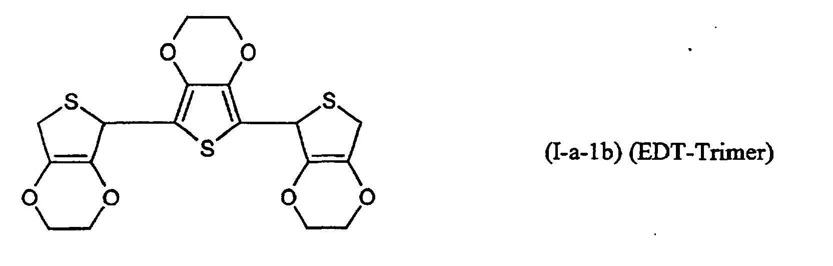 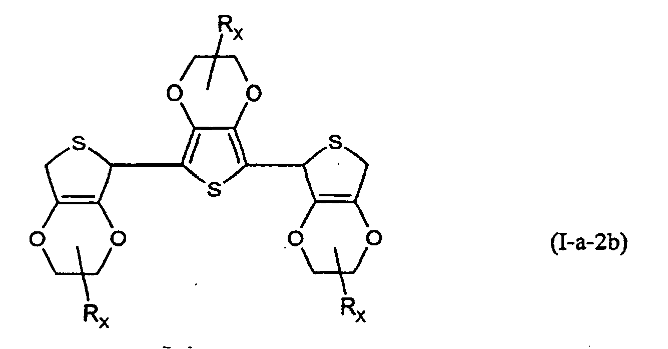 worin
- R: für einen oder mehrere, gleiche oder verschiedene lineare(n) oder verzweigte(n), gegebenenfalls substituierte(n) C₁-C₁₈-Alkylrest(e), bevorzugt lineare(n) oder verzweigte(n), gegebenenfalls substituierte(n) C₁-C₁₄-Alkylrest(e), gegebenenfalls substituierte(n) C₅-C₁₂-Cycloalkylrest(e), gegebenenfalls substituierte(n) C₆-C₁₄-Arylrest(e), gegebenenfalls substituierte(n) C₁-C₄-Hydroxyalkylrest(e), bevorzugt gegebenenfalls substituierte(n) C₁-C₂₋Hydroxyalkylrest(e), oder einen oder mehrere Hydroxylrest(e) steht, und
- x: für eine ganze Zahl von 0 bis 8 steht, bevorzugt von 0 bis 6, besonders bevorzugt für 0 oder 1 steht,
gegebenenfalls im Gemisch mit monomeren Edukten und/oder vorangehend beschriebenen dimeren Produkten der Formel I-a-1a und/oder I-a-2a.

In einer weiteren bevorzugten Ausführungsform sind dies dimere Verbindungen der Formel I-b-1a und I-b-2a, 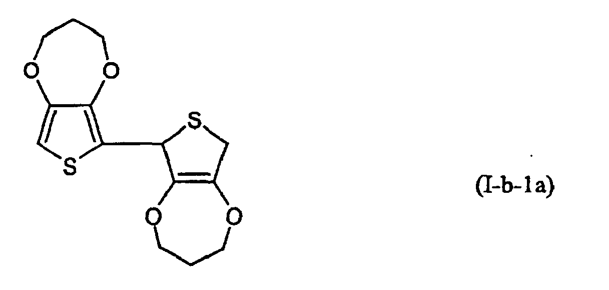 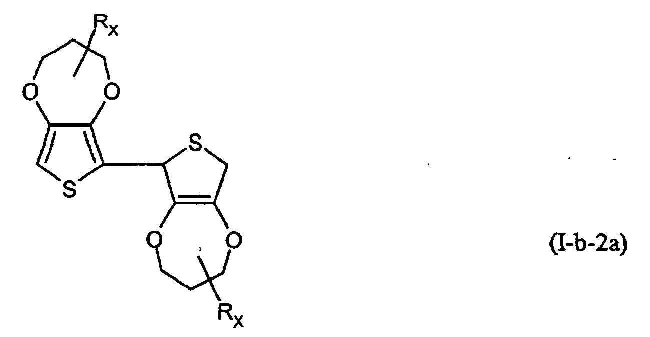 worin
- R: für einen oder mehrere, gleiche oder verschiedene lineare(n) oder verzweigte(n), gegebenenfalls substituierte(n) C₁-C₁₈-Alkyrest(e), bevorzugt lineare(n) oder verzweigte(n), gegebenenfalls substituierte(n) C₁-C₁₄-Alkylrest(e), gegebenenfalls substituierte(n) C₅-C₁₂-Cycloalkylrest(e), gegebenenfalls substituierte(n) C₆-C₁₄-Arylrest(e), gegebenenfalls substituierte(n) C₁-C₄-Hydroxyalkylrest(e), bevorzugt gegebenenfalls substituierte(n) C₁-C₂₋Hydroxyalkylrest(e), oder einen oder mehrere Hydroxylrest(e) steht, und
- x: für eine ganze Zahl von 0 bis 8 steht, bevorzugt von 0 bis 6, besonders bevorzugt für 0 oder 1 steht,
gegebenenfalls im Gemisch mit monomeren Edukten.

In einer weiteren bevorzugten Ausführungsform sind dies trimere Verbindungen der Formel I-b-1b und I-b-2b, 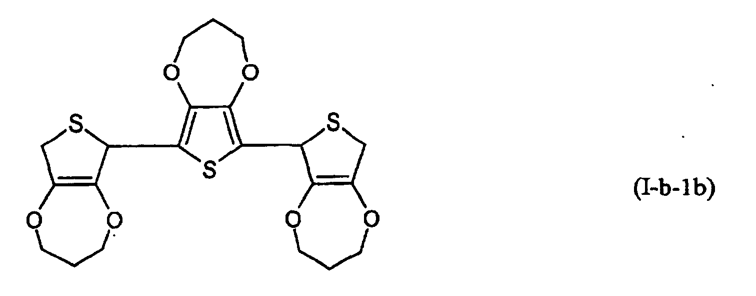 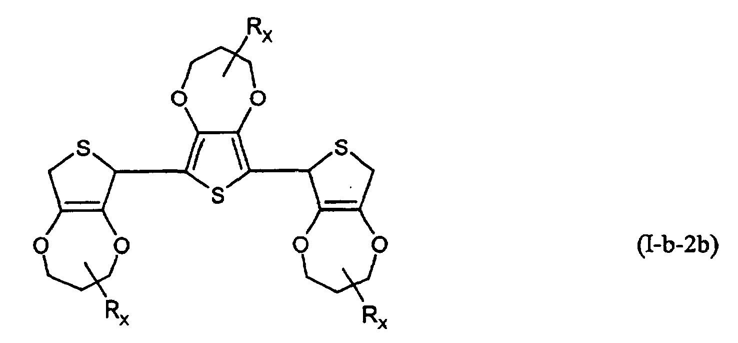 worin
- R: für einen oder mehrere, gleiche oder verschiedene lineare(n) oder verzweigte(n), gegebenenfalls substituierte(n) C₁-C₁₈-Alkylrest(e), bevorzugt lineare(n) oder verzweigte(n), gegebenenfalls substituierte(n) C₁-C₁₄-Alkylrest(e), gegebenenfalls substituierte(n) C₅-C₁₂-Cycloalkylrest(e), gegebenenfalls substituierte(n) C₆-C₁₄-Arylrest(e), gegebenenfalls substituierte(n) C₁-C₄-Hydroxyalkylrest(e), bevorzugt gegebenenfalls substituierte(n) C₁-C₂₋Hydroxyalkylrest(e), oder einen oder mehrere Hydroxylrest(e) steht, und
- x: für eine ganze Zahl von 0 bis 8 steht, bevorzugt von 0 bis 6, besonders bevorzugt für 0 oder 1 steht,
gegebenenfalls im Gemisch mit monomeren Edukten und/oder vorangehend beschriebenen dimeren Produkten der Formel I-b-1a und/oder I-b-2a.

C₁-C₄-Alkylenreste A sind im Rahmen der Erfindung Methylen, Ethylen, n-Propylen oder n-Butylen. Lineare oder verzweigte C₁-C₁₄-Akylreste sind im Rahmen der Erfindung beispielsweise Methyl, Ethyl, n- oder iso-Propyl, n-, iso-, sec- oder tert-Butyl, n-Pentyl, 1-Methylbutyl, 2-Methylbutyl, 3-Methylbutyl, 1-Ethylpropyl, 1,1-Dimethylpropyl, 1,2-Dimethylpropyl, 2,2-Dimethylpropyl, n-Hexyl, n-Heptyl, n-Octyl, 2-Ethylhexyl, n-Nonyl, n-Decyl, n-Undecyl, n-Dodecyl, n-Tridecyl oder n-Tetradecyl, C₁-C₁₈-Alkylreste darüberhinaus n-Hexadecyl oder n-Octadecyl, C₅-C₁₂₋Cycloalkylreste sind beispielsweise Cyclopentyl, Cyclohexyl, Cycloheptyl, Cyclooctyl, Cyclononyl oder Cyclodecyl, C₆-C₁₄-Arylreste sind beispielsweise Phenyl, o-, m-, p-Tolyl, Benzyl, 2,3-, 2,4-, 2,5-, 2,6-, 3,4-, 3,5-Xylyl, Mesityl oder Naphthyl, und C₁-C₄-Hydroxyalkylreste sind beispielsweise Hydroxymethyl, 1-Hydroxyethyl, 2-Hydroxyethyl, 1-Hydroxypropyl, 2-Hydroxypropyl, 3-Hydroxypropyl, 1-Hydroxybutyl, 2-Hydroxybutyl, 3-Hydroxybutyl, 4-Hydroxybutyl. Die vorangehende Aufzählung dient der beispielhaften Erläuterung der Erfindung und ist nicht als abschließend zu betrachten.

Als. Substituenten der oben genannten Reste kommen zahlreiche organische Gruppen in Frage, beispielsweise Halogen-, insbesondere Fluor, Chlor oder Brom, sowie Ether-, Thioether-, Disulfid-, Sulfoxid-, Sulfon-, Aldehyd-, Keto-, Carbonsäureester-, Carbonat-, Cyano-, Alkylsilan- und Alkoxysilangruppen und/oder Carboxylamidgruppen.

Überraschend geschieht die Herstellung der erfindungsgemäßen Verbindungen auf einfache Weise durch Umsetzung von Verbindungen oder Mischungen aus Verbindungen der allgemeinen Formel II 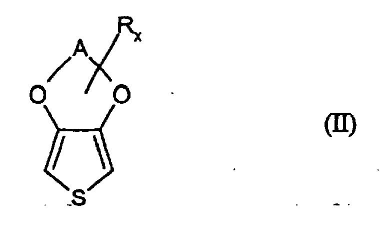 miteinander in Gegenwart einer Lewis-Säure und/oder einer Protonensäure als Katalysator.

Gegenstand der Erfindung ist somit weiterhin ein Verfahren zur Herstellung von Verbindungen der allgemeinen Formel I, 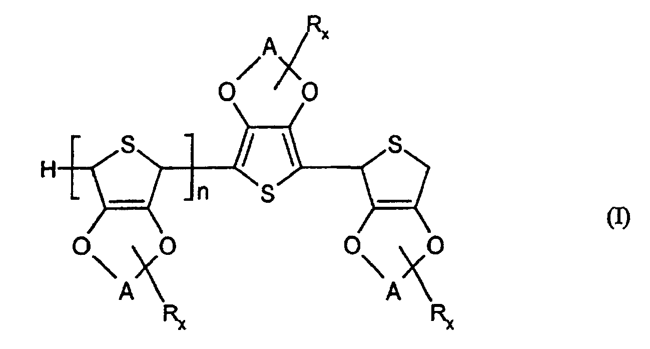 worin
- A, R, x und n: die oben für die allgemeine Formel I genannte Bedeutung haben,
dadurch gekennzeichnet, dass Verbindungen der allgemeinen Formel II, 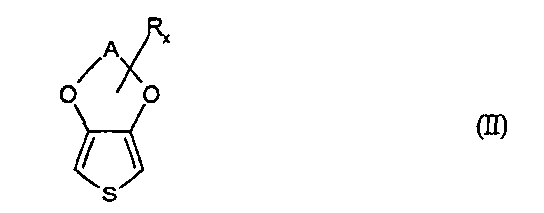 worin
- A, R und x: die für die allgemeine Formel I genannte Bedeutung haben,
in Gegenwart von Lewis-Säuren und/oder Protonensäuren als Katalysator miteinander umgesetzt werden.

Bei der Ausübung des erfindungsgemäßen Verfahrens können auch Gemische aus den monomeren Verbindungen der allgemeinen Formel II mit den erfindungsgemäßen Verbindungen der allgemeinen Formel I entstehen, welche ebenfalls Gegenstand der Erfindung sind. Gegebenenfalls können dies auch Isomerengemische, Racemate sowie reine Isomeren der erfindungsgemäßen Verbindungen gegebenenfalls im Gemisch mit den monomeren Verbindungen der Formel II sein. Eine Aufreinigung dieser Gemische kann mithilfe üblicher Trennverfahren, beispielsweise durch Säulenchromatographie oder fraktionierende Kristallisation sowie Destillation erfolgen. Die säulenchromatographische Trennung an Silicagel und nachfolgende fraktionierende Kristallisation wird bei der Trennung von Edukt/Produkt- und Isomerengemischen der erfindungsgemäßen Verbindungen bevorzugt angewandt. Als Laufmittel zur säulenchromatographischen Trennung werden beispielsweise halogenierte aliphatische Kohlenwasserstoffe, wie z.B. Methylenchlorid, Chloroform, sowie Mischungen aus diesen, bevorzugt Methylenchlorid, eingesetzt.

In bevorzugten Ausführungsformen des erfindungsgemäßen Verfahrens handelt es sich bei Verbindungen der Formel II um die monomeren Verbindungen II-a bis II-d oder Mischungen aus diesen 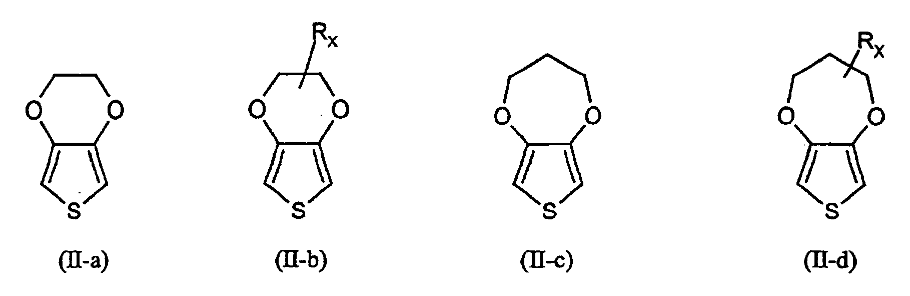 wobei R und x die oben genannte Bedeutung haben.

In einer bevorzugten Ausführungsform wird das erfindungsgemäße Verfahren zur Herstellung von Verbindungen der allgemeinen Formel I in Gegenwart von Lewis-Säuren, besonders bevorzugt nicht-oxidierenden Lewis-Säuren als Katalysatoren durchgeführt.

Bevorzugte Lewissäuren, welche als Katalysatoren im erfindungsgemäßen Verfahren eingesetzt werden, sind Bor- oder Aluminiumtrihalogenide, Phosphortrihalogenide, Titan- oder Zirkoniumtetrahalogenide, Zinn(IV)halogenide, Arsen- und Antimonhalogenide, Tantalpentahalogenide oder Zinkhalogenide, insbesondere bevorzugt Bortrifluorid, Antimonpentachlorid, Aluminiumtrichlorid, Titantetrachlorid, Zinntetrachlorid und Zinkchlorid. Ganz besonders bevorzugt werden Bortrifluorid (z.B. in Form des Etherats oder eines anderen BF₃-Komplexes, z.B. mit Tetrahydrofuran), Antimonpentachlorid, Zinntetrachlorid und Titantetrachlorid eingesetzt.

In einer weiteren bevorzugten Ausführungsform wird das erfindungsgemäße Verfahren zur Herstellung von Verbindungen der allgemeinen Formel I in Gegenwart von Protonensäuren als Katalysatoren durchgeführt.

Bevorzugte Protonensäuren, welche als Katalysatoren im erfindungsgemäßen Verfahren eingesetzt werden, sind Sulfonsäuren, wie Benzolsulfonsäure, p-Toluolsulfonsäure, Methansulfonsäure, Camphersulfonsäure und Poly(styrolsulfonsäure), Carbonsäuren, wie Trifluoressigsäure und Trichloressigsäure, anorganische Säuren, wie HCl, Schwefelsäure und Phosphorsäure, und Supersäuren. Unter Supersäuren sind solche Protonensäuren zu verstehen, deren Acidität größer ist als die von 100%iger Schwefelsäure (Gillespie, Adv. Phys. Org. Chem. **9**, 1- 24 (1972)). Solche Säuren werden z.B. in P.-L- Fabre, J. Devynck und B. Trémillon, Chem. Rev. **82**, 591 - 614 (1982), in G. A. Olah, Angew. Chem. **85,** 183 - 225 (1973) oder in G. A. Olah, Top. Curr. Chem. **80**, 19 - 88 (1979) benannt. Beispiele für Supersäuren sind "Magische Säure" FSO₃H ^{.} SbF₅, FSO₃H ^{.} SbF₅ ^{.} SO₂, HSbF₆, HBF₄, H[B(OSO₃H)]₄, HTaF₆, HF ^{.} SnCl₄.

Besonders bevorzugt werden Protonensäuren ausgewählt aus der Gruppe der Sulfon- oder Carbonsäuren oder Supersäuren, ganz besonders bevorzugt p-Toluolsulfonsäure, Methansulfonsäure, Campher-10-sulfonsäure oder Trifluoressigsäure als Katalysatoren im erfindungsgemäßen Verfahren eingesetzt.

Die bei dem erfindungsgemäßen Verfahren eingesetzten Mengen an Lewis-Säuren und Protonensäuren liegen zwischen 0,01 und 100 Gew.-% Säure, bezogen auf das zu di- und trimerisierende Thiophenmonomer. Dabei liegen die Mengen für Lewissäuren eher im unteren Gewichts-%-Bereich, die für Protonensäuren eher in einem höheren Gewichts-%-Bereich. Daher werden bevorzugt 0,01 bis 50 Gew.-% Lewissäure oder 0,5 bis 80 Gew-% Protonensäure, besonders bevorzugt 0,1 bis 30 Gew.-% Lewissäure oder 1 bis 50 Gew.-% Protonensäure eingesetzt.

Geeignete Lösungsmittel für das erfindungsgemäße Verfahren sind bei Verwendung von Lewissäuren als Katalysatoren z.B. halogenierte aliphatische Kohlenwasserstoffe, wie z.B. Methylenchlorid oder Chloroform, bei Verwendung von Protonensäuren als Katalysatoren je nach Löslichkeit der Protonensäure ebenfalls halogenierte aliphatische Kohlenwasserstoffe oder Alkohole, wie z.B. Methanol, Ethanol oder n-Butanol, sowie Aromaten wie z.B. Toluol, Xylol oder Chlorbenzol. Es ist auch möglich, beispielsweise bei Umsetzung flüssiger Thiophen-Monomeren der allgemeinen Formel II, ganz auf Lösungsmittel zu verzichten.

Unter Umsetzung ist im Sinne der Erfindung vorangehend und im Folgenden die erfindungsgemäße Umsetzung der Verbindungen der allgemeinen Formel II miteinander in Gegenwart einer Lewis-Säure und/oder Protonensäure als Katalysator zu den erfindungsgemäßen Verbindungen der allgemeinen Formel I zu verstehen.

Die Umsetzung zu den erfindungsgemäßen Verbindungen der allgemeinen Formel I kann bei unterschiedlichen Temperaturen durchgeführt werden, wobei die Ausbeuten in Abhängigkeit vom umzusetzenden Thiophen-Monomeren der allgemeinen Formel II unterschiedlich von der Temperatur abhängen können. Im Allgemeinen wird die Umsetzung bei Temperaturen von -30°C bis 100°C, bevorzugt von -5 bis 60°C, besonders bevorzugt von 0°C bis 40°C durchgeführt.

Das erfindungsgemäße Verfahren kann unter Luft oder unter Schutzgas, z.B. Stickstoff oder Argon, durchgeführt werden. Zur Steigerung der Ausbeuten und Reinheiten ist wegen der Vermeidung oxidativer Nebenreaktionen die Anwendung von Schutzgas bevorzugt.

Die Reaktionszeiten sind von der Temperatur und der umzusetzenden Verbindung der allgemeinen Formel II abhängig. Da es sich bei der Umsetzung zu den erfindungsgemäßen Verbindungen je nach eingesetztem Katalysator um eine Gleichgewtchtsreaktion handelt, kann es besonders vorteilhaft sein, durch ein geeignetes Analysenverfahren, beispielsweise ¹H-NMR-Spektroskopie, die im Reaktionsgemisch vorliegenden Anteile an erfindungsgemäßen Verbindungen zu verfolgen und die Reaktion zum günstigsten Zeitpunkt abzubrechen. Ein Abbruch der Umsetzung kann beispielsweise mittels Zugabe von Basen, bevorzugt wässriger Na₂CO₃-Lösung, erfolgen oder mittels Zugabe von Alkoholen, bevorzugt Ethanol, und nachfolgender bzw. gleichzeitiger Zugabe von Wasser oder durch alleinige Zugabe von Wasser herbeigeführt werden.

Im Folgenden werden Beispiele für monomere Verbindungen der allgemeinen Formel II genannt, die zu den erfindungsgemäßen Verbindungen der allgemeinen Formel I-a umgesetzt werden können:

2,3-Dihydrothieno[3,4-b][1,4]dioxin (im Folgenden EDT oder Ethylen-3,4-dioxy thiophen, Verbindung II-a), 2-Methyl-2,3-dihydrothieno[3,4-b][1,4]dioxin (2-Methyl-EDT, Verbindung II-b mit R = Methyl und x = 1), 2-Ethyl-2,3-dihydrothieno[3,4-b][1,4]dioxin (2-Ethyl-EDT, Verbindung II-b mit R = Ethyl und x = 1), 2-n-Propyl-2,3-dihydrothieno[3,4-b][1,4]dioxin (2-n-Propyl-EDT, Verbindung II-b mit R = n-Propyl und x = 1), 2-n-Butyl-2,3-dihydrothieno[3,4-b][1,4]dioxin (2-n-Butyl-EDT, Verbindung II-b mit R = n-Butyl und x = 1), 2-n-Pentyl-2,3-dihydrothieno[3,4-b][1,4]dioxin (2-n-Pentyl-EDT, Verbindung II-b mit R = n-Pentyl und x = 1), 2-n-Hexyl-2,3-dihydrothieno[3,4-b][1,4]dioxin (2-n-Hexyl-EDT, Verbindung II-b mit R = n-Hexyl und x = 1), 2-n-Heptyl-2,3-dihydrothieno[3,4-b][1,4]dioxin (2-n-Heptyl-EDT, Verbindung II-b mit R = n-Heptyl und x = 1), 2-n-Octyl-2,3-dihydrothieno[3,4-b][1,4]dioxin (2-n-Octyl-EDT, Verbindung II-b mit R = n-Octyl und x = 1), 2-(2-Ethylhexyl)-2,3-dihydrothieno[3,4-b][1,4]dioxin (2-(2-Ethylhexyl)-EDT, Verbindung II-b mit R = 2-Ethylhexyl und x = 1), 2-n-Nonyl-2,3-dihydrothieno[3,4-b][1,4]dioxin (2-n-Nonyl-EDT, Verbindung II-b mit R = n-Nonyl und x = 1), 2-n-Tetradecyl-2,3-dihydrothieno[3,4-b][1,4]dioxin (2-Tetradecyl-EDT, Verbindung II-b mit R = n-Tetradecyl und x = 1), 2,3-Dihydrothieno[3,4-b][1,4]dioxin-2-ylmethanol (Verbindung II-b mit R = CH₂OH und x = 1).

Bevorzugte Beispiele aus dieser Gruppe sind 2,3-Dihydrothieno[3,4-b][1,4]dioxin (EDT), 2-Methyl-2,3-dihydrothieno[3,4-b][1,4]dioxin (2-Methyl-EDT) und 2,3-Dihydrothieno[3,4-b][1,4]dioxin-2-ylmethanol.

Ein besonders bevorzugtes Beispiel aus dieser Gruppe ist 2,3-Dihydrothieno[3,4-b][1,4]dioxin (EDT).

Im Folgenden werden Beispiele für monomere Verbindungen der allgemeinen Formel II genannt, die zu den erfindungsgemäßen Verbindungen der allgemeinen Formel I-b umgesetzt werden können:

3,4-Dihydro-2H-thieno[3,4-b][1,4]dioxepin (Propylen-3,4-dioxythiophen, PDT, Verbindung II-c), 3-Methyl-3,4-dihydro-2H-thieno[3,4-b][1,4]dioxepin (3-Methyl-PDT, Verbindung II-d mit R = Methyl und x = 1), 3,3-Dimethyl-3,4-dihydro-2H-thieno[3,4-b][1,4]dioxepin (3,3-Dimethyl-PDT, Verbindung II-d mit R = Methyl und x = 2), 2-Methyl-3,4-dihydro-2H-thieno[3,4-b][1,4]dioxepin (2-Methyl-PDT, Verbindung II-d mit R = Methyl und x = 1), 3,4-dihydro-2H-thieno[3,4-b][1,4]dioxepin-3-ol, (Verbindung II-d mit R = OH und x =1).

Bevorzugte Beispiele aus dieser Gruppe sind 3,4-Dihydro-2H-thieno[3,4-b][1,4]-dioxepin (PDT) und 3,4-Dihydro-2H-thieno[3,4-b][1,4]dioxepin-3-ol.

Alle angegebenen Verbindungen können auch im Gemisch miteinander eingesetzt werden, wobei Gemische unterschiedlicher Di- und Trimerer ebenso entstehen können wie gemischte Dimere und Trimere aus verschiedenen Monomerbausteinen sowie Gemische aus beiden Verbindungstypen (d. h., aus denen mit einheitlichen Monomerbausteinen und denen, die aus verschiedenen Monomerbausteinen zusammengesetzt sind).

Die Herstellung der neuen, erfindungsgemäßen Verbindungen und das erfindungsgemäße Verfahren zur Herstellung ist ausgesprochen überraschend, besonders da in A. K. Mohanakrishnan, A. Hucke, M. A. Lyon, M. V. Lakshmikantham und M. P. Cava, Tetrahedron 55 (1999), S. 11745 - 11754 dargestellt wird, dass z.B. Ethylen-3,4-dioxythiophen (EDT, Verbindung II-a) unter stark sauren Bedingungen oder in Gegenwart von Lewis-Säuren umfangreiche Zersetzungsreaktionen eingeht.

Die erfindungsgemäßen Verbindungen der allgemeinen Formel I können chemisch oder elektrochemisch oxidativ zu Polythiophenen umgesetzt werden. Als chemische Oxidationsmittel sind die aus dem Stand der Technik zur Polythiophenherstellung bekannten geeignet, beispielsweise Eisen-III-Verbindungen wie FeCl₃, Eisen-III-tosylat, Kaliumpermanganat oder Braunstein (MnO₂), Kalium(di)chromat, Peroxodisulfate (Persulfate) wie Na₂S₂O₈ oder K₂S₂O₈ sowie H₂O₂.

Weiterhin Gegenstand der Erfindung ist somit die Verwendung der erfindungsgemäßen Verbindungen der allgemeinen Formel I zur Herstellung von neutralen oder kationischen Polythiophenen.

Die Polythiophene können bereits durch das Herstellverfahren halbleitend bis hochleitfähig sein oder aber durch nachträgliche Oxidation diese Eigenschaften erlangen. Im Rahmen der Erfindung werden unter halbleitend solche Polythiophene verstanden, die eine Leitfähigkeit kleiner als 10 S/cm aufweisen, und unter leitfähig bis hochleitfähig solche mit einer Leitfähigkeit gleich oder größer als 10 S/cm, wobei in Abhängigkeit des Substitutionsmusters der Thiophenwiederholungseinheiten, d.h je nach Bedeutung von A, R und x, diese Grenze auch nach oben oder unten verschoben sein kann.

Weiterhin Gegenstand der vorliegenden Erfindung ist somit ein Verfahren zur Herstellung von neutralen oder kationischen Polythiophenen der allgemeinen Formel III, 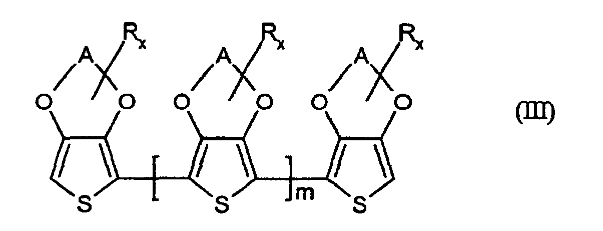 worin
- A, R und x: die oben für die allgemeine Formel I genannte Bedeutung haben und
- m: für eine ganze Zahl von 2 bis 200 steht,
dadurch gekennzeichnet, dass Verbindungen der allgemeinen Formel I chemisch oder elektrochemisch oxidativ polymerisiert werden

Unter dem Begriff Polythiophene sind im Rahmen der Erfindung auch Oligothiophene mit umfasst, so dass Polythiophene alle Verbindungen gemäß der allgemeinen Formel III umfasst, für die m mindestens 2 und höchstens 200 ist.

Die Polythiophene der allgemeinen Formel III können neutral oder kationisch sein. Für den Fall, dass es sich um kationische Polythiophene handelt, werden die positiven Ladungen der Polythiophen-Polykationen nicht in der Formel III dargestellt, da ihre genaue Zahl und ihre Position nicht einwandfrei feststellbar sind. Die Anzahl der positiven Ladungen beträgt jedoch mindestens 1 und höchstens m + 2.

Zur Kompensation der positiven Ladung enthält die kationische Form der Polythiophene Anionen, vorzugsweise Polyanionen, als Gegenionen.

Als Polyanionen dienen vorzugsweise die Anionen von polymeren Carbonsäuren, wie Polyacrylsäuren, Polymethacrylsäure oder Polymaleinsäuren und polymeren Sulfonsäuren, wie Polystyrolsulfonsäuren und Polyvinylsulfonsäuren. Diese Polycarbon- und -sulfonsäuren können auch Copolymere von Vinylcarbon- und Vinylsulfonsäuren mit anderen polymerisierbaren Monomeren, wie Acrylsäureestern und Styrol, sein.

Besonders bevorzugt ist das Anion der Polystyrolsulfonsäure als Gegenion.

Das Molekulargewicht der die Polyanionen liefernden Polysäuren beträgt vorzugsweise 1 000 bis 2 000 000, besonders bevorzugt 2 000 bis 500 000. Die Polysäuren oder ihre Alkalisalze sind im Handel erhältlich, z.B. Polystyrolsulfonsäuren und Polyacrylsäuren.

Wegen der hohen Reaktivität der erfindungsgemäßen Verbindungen verläuft die oxidative Polymerisation besonders leicht und schnell, was vorteilhaft gegenüber aus dem Stand der Technik bekannten oxidativen Polymerisationverfahren anderer Monomeren ist.

Die erfindungsgemäßen Verbindungen der allgemeinen Formel I sowie die daraus hergestellten neutralen und/oder kationischen Polythiophene können zur Herstellung von organischen elektrischen oder elektronischen Bauteilen, z.B. zur Herstellung von Leuchtelementen, Photozellen oder organischen Transistoren, zur Ausrüstung von Kunststofffolien für die Verpackung elektronischer Bauteile und für Reinraumverpackungen, für die antistatische Ausrüstung von Kathodenstrahlröhren, für die antistatische Ausrüstung von photographischen Filmen, als transparente Heizung, als transparente Elektroden, als Leiterplatten oder für elektrisch einfärbbare Fensterscheiben dienen. Durch oxidative Polymerisation ist es zum Beispiel möglich, leitfähige Schichten auf nicht leitenden Substraten, wie Glas, Keramik, Kunststoff etc., zu erzeugen. In Kondensatoren können die so erzeugten Schichten die Rolle der Kathode übernehmen.

Dabei können die erfindungsgemäßen Verbindungen der allgemeinen Formel I im Gemisch mit dem Oxidationsmittel aus organischen Lösungsmitteln, wie beispielsweise Alkoholen, Methylenchlorid, Chloroform, N-Methylpyrrolidon etc., durch Aufrakeln, spin coating, Gießen, Tränken, etc. auf die Substrate aufgebracht werden.

Die nach dem erfindungsgemäßen Verfahren hergestellten bzw. erfindungsgemäßen Verbindungen der allgemeinen Formel I sind oft hellgelbliche Öle oder beige Feststoffe, die in organischen Lösungsmitteln wie Methylenchlorid, Chloroform oder Tetrahydrofuran löslich sind. Sie sind daher für Anwendungen, z.B. in der Elektronikindustrie, aus organischer Lösung leicht verarbeitbar. Aus solchen Lösungen lassen sich in Anwesenheit eines Gegenions leicht, d. h. auch mit milden Oxidationsmitteln, hervorragend leitfähige kationische Polythiophene bzw. Polythiophen-Schichten herstellen.

Weiterhin Gegenstand der vorliegenden Erfindung ist daher die Verwendung der Verbindungen der allgemeinen Formel I zur Herstellung von Bestandteilen elektrischer oder elektronischer Bauteile.

Weiterhin erfindungsgemäß ist die Verwendung der Verbindungen der allgemeinen Formel III, welche nach dem oben genannten oxidativen Polymerisationsverfahren aus den erfindungsgemäßen Verbindungen der allgemeinen Formel I hergestellt wurden, als Bestandteil von elektrischen und elektronischen Bauteilen, insbesondere als Kathoden in Kondensatoren. Beispielsweise können sie als Kathoden in Kondensatoren verwendet werden, welche Anoden aus Ventilmetallen, z.B. Aluminium, Niob, Tantal oder Legierungen aus diesen, enthalten.

### Beispiele

Die im Folgenden verwendeteten Edukte (EDT und alkylsubstituierte EDT-Derivate) sind entweder käuflich zu erwerben oder können durch Umetherung der entsprechenden 3,4-Dialkoxythiophene (beispielsweise 3,4-Di-n-propoxythiophen), welche nach bekannten Verfahren leicht zugänglich sind, mit vicinalen 1,2-Diolen hergestellt werden.

### Allgemeine Vorschrift zur Synthese der alkylsubstituierten EDT-Derivate:

Beispielhaft kann die Synthese der alkylsubstituierten EDT-Derivate durch Umetherung so erfolgen, dass 3,4-Di-n-propoxythiophen und ein geminales 1,2-Diol im Mol-Verhältnis 1 : 2 (100 % Überschuss des Diols) mit p-Toluolsulfonsäure als Katalysator in Toluol unter N₂ 2 h zum Rückfluß erhitzt werden, anschließend Toluol/n-Propanol langsam abdestilliert und kontinuierlich durch Xylol ersetzt werden. Die Reaktion wird so lange fortgeführt, bis das abdestillierte Xylol kein n-Propanol mehr enthält (Kontrolle z. B. über den Brechungsindex) oder dünnschichtchromatographisch kein 3,4-Di-n-propoxythiophen mehr nachzuweisen ist. Gegebenenfalls wird abdestilliertes Xylol kontinuierlich durch frisches ersetzt. Nach Abkühlen der Lösung wird mit Wasser neutral gewaschen, über Na₂SO₄ getrocknet und an einer Kieselgel-Säule mit Toluol/n-Hexan (1 : 1) chromatographiert. Nach Entfernen des Lösungsmittels erhält man das alkylsubstituierte EDT-Derivat.

### Beispiel 1

### Herstellung von 2,2',3,3',5,7-Hexahydro-5,5'-bithieno[3,4-b][1,4]dioxin (I-a-1a, EDT-Dimer) und 2,2',2",3,3',3",5,5",7,7"-Decahydro-5,5':7',5"-terthieno[3,4-b][1,4]-dioxin (I-a-1b, EDT-Trimer) mit BF₃ als Katalysator

Zu einer Lösung von 0,284 g (2 mmol) 2,3-Dihydrothieno[3,4-b][1,4]dioxin (Ethylen-3,4-dioxythiophen oder EDT; Baytron® M, H. C. Starck GmbH) in 1,5 ml.

Methylenchlorid wurden 2 ml einer Lösung von 0,04 ml BF₃-Etherat in 20 ml Methylenchlorid (entsprechend 0,036 mmol BF₃ bzw. 1,8 Gew.-% Bortrifluorid-Etherat, bez. auf EDT) bei 0°C zugegeben. Das Reaktionsgemisch wurde 4 h unter Ar-Atmosphäre bei 0°C gerührt. Danach wurde die Reaktion durch Zugabe von 2 ml Ethanol und anschließend 4 ml Wasser gestoppt. Die organische Phase wurde abgetrennt und am Rotationsverdampfer bei 20 mbar vom Lösungsmittel befreit. Der Rückstand wurde an Silicagel säulenchromatographisch mit Methylenchlorid als Laufmittel aufgetrennt.
Fraktion 1: 0,128 g EDT (45,5 % des eingesetzten Edukts)
Fraktion 2: 0,045 g (16 % d. Th.) 2,2',3,3',5,7-Hexahydro-5,5'-bithieno[3,4-b][1,4]dioxin (EDT-Dimer) als Racemat.

| | |
|---|---|
| Elementaranalyse: | Ber.: C 50,69 %; H 4,25 %; S 22,55 % (C₁₂H₁₂O₄S₂) |
| | Gef.: C 50,49 %; H 4,45 %; S 22,59 % |

Massenspektrum (70 eV); m/z, rel. Intensität): 284 (100,0 %), M⁺; 251 (66,7 %); 223 (11,0 %); 195 (27,9 %); 186 (18,4 %); 185 ( 15,0 %); 168 (11,7 %); 155 (15,4 %); 154 (19,2 %); 143 (4,0 %), EDT-H⁺; 142 (14,9 %), EDT⁺
¹H-NMR (CDCl₃, δ in ppm, *J* in Hz): δ: 3,60 (dd, 1H, ²*J* = 11,93, ⁵*J* = 1,96); 3,80 (dd, 1H, ²*J* = 11,93, ^{5'}*J* = 5,48); 4,08 (m, 4H); 4,18 (m, 4H); 5,44 (dd, 1H, ⁵*J* = 1,96, ^{5'}*J* = 5,48); 6,26 (s, 1H);
¹³C{¹H}NMR (C₆D₆, δ in ppm): δ: 30,54; 42,42; 64,28; 64,39; 98,27; 120,58; 129,89; 130,68; 139,69; 141,70.
Fraktion 3: 0,018 g (6 % d. Th.) 2,2',2",3,3',3",5,5",7,7"-Decahydro-5,5':7',5"-terthieno[3,4-b][1,4]dioxin (EDT-Trimer), Stereoisomeren-Mischung.
Massenspektrum (70 eV); m/z, rel. Intensität): 426 (54,8 %), M⁺; 394 (22,2 %), M⁺⁻S; 393 (22,8 %); 284 (88,2 %), M⁺-EDT; 283 (64,4 %); 282 (93,5 %); 251 842,0 %); 223 (6,9 %); 195 (15,7 %); 186 (16,4 %); 185 (80,6 %); 181 (7,1 %); 168 (7,6 %); 155 (14,0 %); 153 (8,9 %); 142 (73,8 %), EDT⁺.
¹³C{¹H}NMR (C₆D₆, δ in ppm): δ: 30,53; 30,60; 42,47; 42,53; 64,11; 64,18; 64,20; 64,24; 64,35; 118,93; 119,19; 129,71; 129,79; 130,59; 130,67; 138,04; 139,02.

Umkristallisation von Fraktion 3 aus Hexan/Diethylether/Methylenchlorid-Gemisch ergab 3 mg des R,S-Isomers des EDT-Trimers:
¹H-NMR (CDCl₃, δ in ppm, J in Hz): δ: 3,59 (dd, 2H, ^{*2*}*J* = 11,94, ⁵*J* = 1,96); 3,77 (dd, 2H, ^{*2*}*J* = 11,94, ^{*5*}*J* = 5,48); 4,08 (m, 8H), 4,18 (br. s, 4H); 5,43 (dd, 2H, ⁵*J* = 1,96, ^{5'}*J* = 5,48).

Aus der Mutterlauge dieser Umkristallisation wurden durch Eindampfen 14 mg einer angereicherten Probe des R,R/S,S-Isomerengemisches des EDT-Trimers erhalten.
¹H-NMR (CDCl₃, δ in ppm, J in Hz) der Hauptkomponente: δ: 3,58 (dd, 2H, ^{*2*}*J* = 12,08, ⁵*J* = 1,59); 3,78 (dd, 2H, ²*J* = 12,00, ^{5'}*J* = 5,40); 4,07 (m, 8H), 4,18 (m, 4H); 5,42 (dd, 2H, ⁵*J* = 1,43, ^{*5'*}*J* = 5,40).

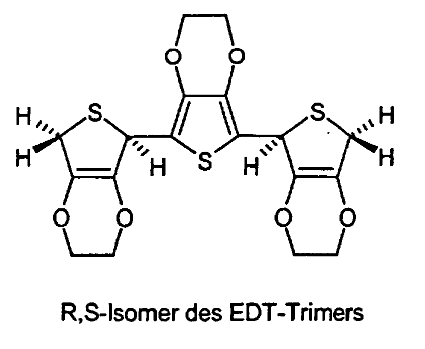

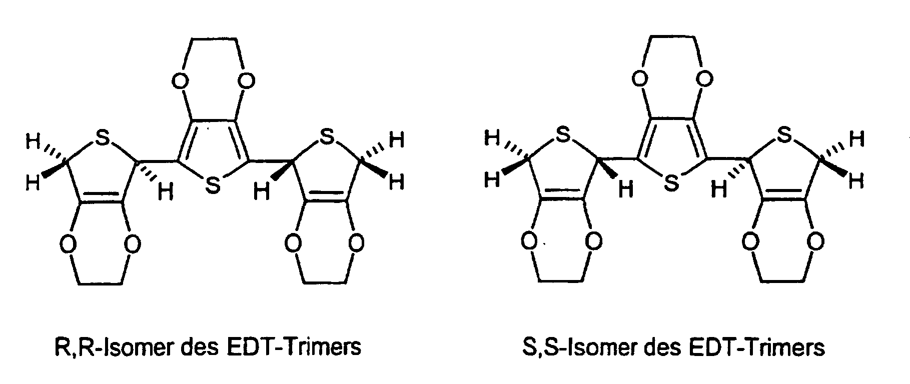

### Beispiel 2

### Herstellung von EDT-Dimer und EDT-Trimer mit Trifluoressigsäure als Katalysator

Zu einer Lösung von 8,52 g (0,06 mol) Ethylen-3,4-dioxythiophen (EDT) in 13,5 ml Methylenchlorid wurden 0,426 g Trifluoressigsäure in 18 ml Methylenchlorid bei 18°C zugegeben. Das Reaktionsgemisch wurde 48 h unter N₂-Atmosphäre bei 18°C gerührt. Danach wurde die Reaktion durch intensives Rühren mit 30 ml 5 %iger wässriger Na₂CO₃-Lösung gestoppt. Die organische Phase wurde abgetrennt, mit Wasser neutral gewaschen und am Rotationsverdampfer bei 20 mbar vom Lösungsmittel befreit. Der Rückstand (8,45 g) wurde an Silicagel säulenchromatographisch mit Methylenchlorid als Laufmittel aufgetrennt.
Fraktion 1: 4,21 g EDT (49,4 % des eingesetzten Edukts)
Fraktion 2: 2,22 g (26,1 % d. Th.) EDT-Dimer, identifiziert mittels ¹H-NMR in CDCl₃. Das Spektrum ist identisch mit dem in Beispiel 1 beschriebenen. Beige Kristalle,
   Schmp. 115-119°C.
Fraktion 3: 1,0 g (11,7 % d. Th.) EDT-Trimer als Isomerengemisch, identifiziert mittels ¹H-NMR in CDCl₃. Das Spektrum entspricht einer Überlagerung der in Beispiel 1 angegebenen Daten für die R,S- und R,R/S,S-Isomeren. Beige Kristalle.

### Beispiel 3

### Herstellung von EDT-Dimer und EDT-Trimer mit TiCl₄ als Katalysator

Zu einer Lösung von 8,52 g (0,06 mol) Ethylen-3,4-dioxythiophen (EDT) in 13,5 ml Methylenchlorid wurden 0,426 g Titantetrachlorid in 18 ml Methylenchlorid bei 20°C zugegeben. Das Reaktionsgemisch wurde 8 h unter N₂-Atmosphäre bei 20°C gerührt. Danach wurde die Reaktion durch intensives Rühren mit 30 ml 5 %iger wässriger Na₂CO₃-Lösung gestoppt. Die organische Phase wurde mit 20 ml Methylenchlorid verdünnt und abgetrennt, mit Wasser neutral gewaschen und am Rotationsverdampfer bei 20 mbar vom Lösungsmittel befreit. Der Rückstand (8,0 g) wurde an Silicagel säulenchromatographisch mit Methylenchlorid als Laufmittel aufgetrennt.
Fraktion 1: im Wesentlichen EDT
Fraktion 2: 1,37 g (16,1 % d. Th.) EDT-Dimer, identifiziert mittels ¹H-NMR in CDCl₃. Das Spektrum ist identisch mit dem in Beispiel 1 beschriebenen. Beige Kristalle,
   Schmp.113-114°C.
Fraktion 3: 0,96 g (11,3 % d. Th.) EDT-Trimer als Isomerengemisch (kleiner Anteil Dimer enthalten), identifiziert mittels ¹H-NMR in CDCl₃: Das Spektrum entspricht einer Überlagerung der in Beispiel 1 angegebenen Daten für die R,S- und R,R/S,S-Isomeren. Beige Kristalle.

### Beispiel 4

### Herstellung der Verbindung der Formel I-a-2a mit R = Methyl und x = 1 (2-Methyl-EDT-Dimer) und der Verbindung der Formel I-a-2b mit R = Methyl und x = 1 (2-Methyl-EDT-Trimer) mit BF₃ als Katalysator

Zu einer Lösung von 3,124 g (20 mmol) 2-Methyl-2,3-dihydrothieno[3,4-b][1,4]dioxin (2-Methyl-EDT; hergestellt gemäß oben genannter Synthesevorschrift) in 15 ml Methylenchlorid wurden 0,12 ml BF₃-Etherat in 20 ml Methylenchlorid (entsprechend 0,95 mmol BF₃ bzw. 4,3 Gew.-% BF₃-Etherat, bezogen auf Methyl-EDT) bei 0°C zugegeben. Das Reaktionsgemisch wurde 4 h unter N₂-Atmosphäre bei 0 bis 2°C gerührt. Danach wurde die Reaktion durch Zugabe von 20 ml Ethanol und anschließend 40 ml Wasser gestoppt. Die organische Phase wurde abgetrennt und am Rotationsverdampfer bei 20 mbar vom Lösungsmittel befreit. Der Rückstand wurde an Silicagel säulenchromatographisch mit Methylenchlorid als Laufmittel aufgetrennt.
Fraktion 1: 1,34 g Edukt (42,9 % des eingesetzten 2-Methyl-EDT)
Fraktion 2: 0,79 g (25,3 % d. Th.) 2-Methyl-EDT-Dimer, Isomerengemisch, identifiziert mittels ¹H-NMR:
   ¹H-NMR (CDCl₃, δ in ppm): δ: 1,25 - 1,34 (mehrere s, 3H); 3,55 - 3,80 (m, 2H); 3,8 - 3,9 (m, 2H); 4,0 - 4,35 (m, 4H); 5,40 - 5,47 (m, 1H); 6,24 (mehrere dicht beieinander liegende s, 1H);
Fraktion 3: 0,34 g (10,9 % d. Th.) 2-Methyl-EDT-Trimer, nach ¹H-NMR (CDCl₃) verunreinigt mit etwas 2-Methyl-EDT-Dimer.

### Beispiel 5

### Herstellung der Verbindung der Formel I-a-2a mit R = n-Tetradecyl und x = 1 (2-Tetradecyl-EDT-Dimer) und der Verbindung der Formel I-a-2b mit R = n-Tetradecyl und x = 1 (2-Tetradecyl-EDT-Trimer) mit BF₃ als Katalysator

Zu einer Lösung von 3,386 g (10 mmol) 2-n-Tetradecyl-2,3-dihydrothieno[3,4-b][1,4]dioxin (2-Tetradecyl-EDT; hergestellt gemäß oben genannter Synthesevorschrift) in 15 ml Methylenchlorid wurden 0,06 ml BF₃-Etherat in 20 ml Methylenchlorid (entsprechend 0,47 mmol BF₃ bzw. 2,0 Gew.-% BF₃-Etherat, bezogen auf 2-Tetradecyl-EDT) bei 0°C zugegeben. Das Reaktionsgemisch wurde 4 h unter N₂₋Atmosphäre bei 0°C gerührt. Danach wurde die Reaktion durch Zugabe von 20 ml Ethanol und anschließend 40 ml Wasser gestoppt. Die organische Phase wurde abgetrennt und am Rotationsverdampfer bei 20 mbar vom Lösungsmittel befreit. Es wurden 3,34 Rückstand erhalten, der nach ¹H-NMR (CDCl₃) aus 47,2 Gew.-% 2-Tetradecyl-EDT = Edukt, 42,1 Gew.% 2-Tetradecyl-EDT-Dimer und 10,7 Gew.-% 2-Tetradecyl-EDT-Trimer besteht (Auswertung der Absorptionen bei δ = 6,20 bis 6,24 ppm und 5,37 bis 5,5 ppm).

### Beispiel 6

Umsetzung eines Gemisches aus 80 % 2,3-Dihydrothieno[3,4-b][1,4]dioxin-2-ylmethanol und 20 % 3,4-dihydro-2H-thieno[3,4-b][1,4]dioxepin-3-ol in Gegenwart von BF₃ als Katalysator.

Zu einer Lösung von 3,444 g (20 mmol) eines Gemisches aus 80 % 2,3-Dihydrothieno[3,4-b][1,4]dioxin-2-ylmethanol und 20 % 3,4-Dihydro-2H-thieno[3,4-b][1,4]dioxepin-3-ol (Baytron® M OH-Versuchsprodukt CH 8020, H. C. Starck GmbH) in 45 ml Methylenchlorid wurden 0,12 ml BF₃-Etherat in 60 ml Methylenchlorid (entsprechend 0,95 mmol BF₃ bzw. 1,9 Gew.-% Bortrifluorid-Etherat, bez. auf das Thiophen-Eduktgemisch) bei 0°C zugegeben. Das Reaktionsgemisch wurde 4 h unter N₂-Atmosphäre bei 0°C gerührt. Danach wurde die Reaktion durch Zugabe von 60 ml Ethanol und anschließend 120 ml Wasser gestoppt. Die organische Phase wurde abgetrennt und am Rotationsverdampfer bei 20 mbar vom Lösungsmittel befreit. Es wurden 3,21 g Rückstand erhalten, der nach ¹H-NMR (CDCl₃) grob abgeschätzt aus etwa 55 Gew.-% Edukt, 30 Gew.-% Dimeren und 15 Gew. Trimeren besteht (Auswertung der Absorptionen bei δ = 6,25 bis 6,3 ppm und 5,35 bis 5,55 ppm). Die Struktur einzelner Verbindungen bzw. Addukte kann wegen der Komplexität des Spektrums nicht ermittelt werden. Eine Absorption bei 6,52 ppm spricht für das Vorliegen einer dimeren Struktur aus dem 3,4-Dihydro-2H-thieno[3,4-b][1,4]dioxepin-3-ol (max. 2 Gew.-%).

### Beispiel 7

### Herstellung von EDT-Dimer und EDT-Trimer mit p-Toluolsulfonsäure als Katalysator in Ethanol

2 g (14 mmol) Ethylen-3,4-dioxythiophen (EDT) und 1 g p-Toluolsulfonsäure wurden in 50 ml Ethanol gelöst und bei 20°C 10 Tage unter N₂-Atmosphäre gerührt. Danach wurde die Reaktion Zugabe von 30 ml 5 %iger wässriger Na₂CO₃-Lösung gestoppt. Die Lösung wurde mit Methylenchlorid ausgeschüttelt, die organische Phase mit Wasser neutral gewaschen und am Rotationsverdampfer bei 20 mbar vom Lösungsmittel befreit. Es verblieben 1,65 g Rückstand, der nach ¹H-NMR in CDCl₃ 5,2 Gew.-% des EDT-Dimeren und eine Spur des EDT-Trimeren enthielt (NMR-spektroskopische Daten entsprechend den reinen Verbindungen in Beispiel 1).

### Beispiel 8

### Herstellung von EDT-Dimer und EDT-Trimer mit Methansulfonsäure als Katalysator

Zu einer Lösung von 8,52 g (0,06 mol) Ethylen-3,4-dioxythiophen (EDT) in 135 ml Methylenchlorid wurden 0,426 g Methansulfonsäure in 180 ml Methylenchlorid bei 20°C zugegeben. Das Reaktionsgemisch wurde 6 h unter N₂-Atmosphäre bei 20°C gerührt. Danach wurde die Reaktion durch intensives Rühren mit 30 ml 5 %iger wässriger Na₂CO₃-Lösung gestoppt. Die organische Phase wurde abgetrennt, mit Wasser neutral gewaschen und am Rotationsverdampfer bei 20 mbar vom Lösungsmittel befreit. Der Rückstand (8,5 g) enthielt nach ¹H-NMR in CDCl₃ 6,3 Gew.-% des EDT-Dimeren (NMR-spektroskopische Daten entsprechend den isolierten Verbindungen in Beispiel 1).

### Beispiel 9

### Herstellung von EDT-Dimer und EDT-Trimer mit SnCl₄ als Katalysator

Zu einer Lösung von 8,52 g (0,06 mol) Ethylen-3,4-dioxythiophen (EDT) in 13,5 ml Methylenchlorid wurden 0,426 g Zinn(IV)chlorid in 18 ml Methylenchlorid bei 20°C in 30 min zugetropft. Das Reaktionsgemisch wurde 6 h unter N₂-Atmosphäre bei 20°C gerührt. Danach wurde die Reaktion durch intensives Rühren mit 30 ml 5 %iger wässriger Na₂CO₃-Lösung gestoppt. Die organische Phase wurde mit 20 ml Methylenchlorid verdünnt und abgetrennt, mit Wasser neutral gewaschen und am Rotationsverdampfer bei 20 mbar vom Lösungsmittel befreit. Der Rückstand (6,99 g) bestand nach ¹H-NMR in CDCl₃ aus 60,5 Gew.-% EDT, 34,2 Gew.-% EDT-Dimer und 5,3 Gew.-% EDT-Trimer (NMR-spektroskopische Daten entsprechend den isolierten Verbindungen in Beispiel 1).

### Beispiel 10

### Herstellung von EDT-Dimer und EDT-Trimer mit SbCl₅ als Katalysator

Zu einer Lösung von 8,52 g (0,06 mol) Ethylen-3,4-dioxythiophen (EDT) in 13,5 ml Methylenchlorid wurden 0,296 g Antimon(V)chlorid in 18 ml Methylenchlorid bei 20°C in 30 min zugetropft. Das Reaktionsgemisch wurde 8 h unter N₂-Atmosphäre bei 20°C gerührt. Danach wurde die Reaktion durch intensives Rühren mit 30 ml 5 %iger wässriger Na₂CO₃-Lösung gestoppt. Die organische Phase wurde mit 20 ml Methylenchlorid verdünnt und abgetrennt, mit Wasser neutral gewaschen und am Rotationsverdampfer bei 20 mbar vom Lösungsmittel befreit. Der Rückstand (8,5 g) bestand nach ¹H-NMR in CDCl₃ aus 37,2 Gew.-% EDT, 49,0 Gew.-% EDT-Dimer, 13,8 Gew.-% EDT-Trimer (NMR-spektroskopische Daten entsprechend den isolierten Verbindungen in Beispiel 1).

## Patentansprüche

1. Verbindungen der allgemeinen Formel I, **dadurch gekennzeichnet, dass** 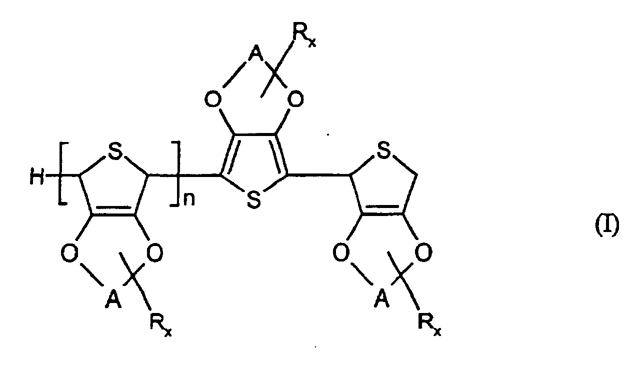
A für einen gegebenenfalls substituierten C₁-C₄-Alkylenrest steht,
R für einen oder mehrere, gleiche oder verschiedene lineare(n) oder verzweigte(n), gegebenenfalls substituierte(n) C₁-C₁₈-Alkylrest(e), gegebenenfalls substituierte(n) C₅-C₁₂-Cycloalkylrest(e), gegebenenfalls substituierte(n) C₆-C₁₄-Arylrest(e), gegebenenfalls substituierte(n) C₁-C₄-Hydroxyalkylrest(e) oder einen oder mehrere Hydroxylrest(e) steht,
x für eine ganze Zahl von 0 bis 8 steht und
n für 0 oder 1 steht.

2. Verbindungen gemäß Anspruch 1, **dadurch gekennzeichnet, dass**
A für einen gegebenenfalls substituierten C₂- oder C₃-Alkylenrest steht,
R für einen oder mehrere, gleiche oder verschiedene lineare(n) oder verzweigte(n), gegebenenfalls substituierte(n) C₁-C₁₈-Alkylrest(e), gegebenenfalls substituierte(n) C₅-C₁₂-Cycloalkylrest(e), gegebenenfalls substituierte(n) C₆-C₁₄-Arykest(e), gegebenenfalls substituierte(n) C₁-C₄-Hydroxyalkylrest(e) oder Hydroxylrest steht,
x für eine ganze Zahl von 0 bis 6 steht und
n für 0 oder 1 steht.

3. Verbindungen gemäß wenigstens einem der Ansprüche 1 oder 2, **dadurch gekennzeichnet, dass**
A für einen gegebenenfalls substituierten C₂- oder C₃-Alkylenrest steht,
R für einen linearen oder verzweigten, gegebenenfalls substituierten C₁₋C₁₄-Alkylrest, einen gegebenenfalls substituierten C₁-C₂-Hydroxy alkykest oder einen Hydroxylrest steht,
x für 0 oder 1 steht und
n für 0 oder 1 steht.

4. Verbindungen gemäß wenigstens einem der Ansprüche 1 bis 3, **dadurch gekennzeichnet, dass** sie eine Struktur der allgemeinen Formeln I-a-1 oder I-b-1 besitzen, 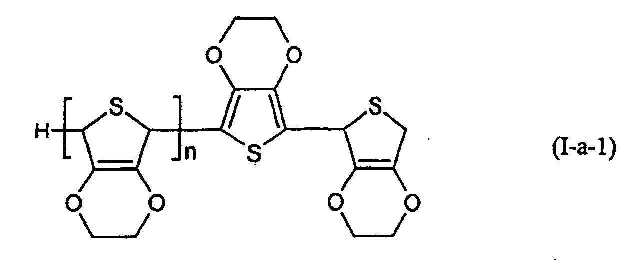 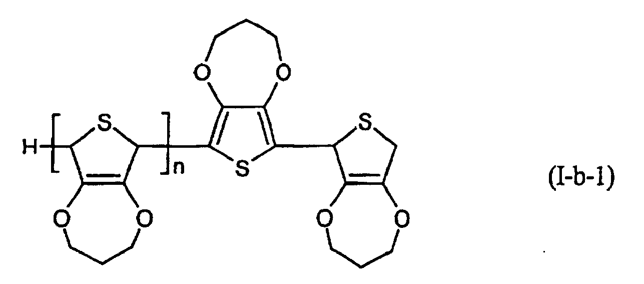 worin
n für 0 oder 1 steht.

5. Verfahren zur Herstellung von Verbindungen der allgemeinen Formel I, 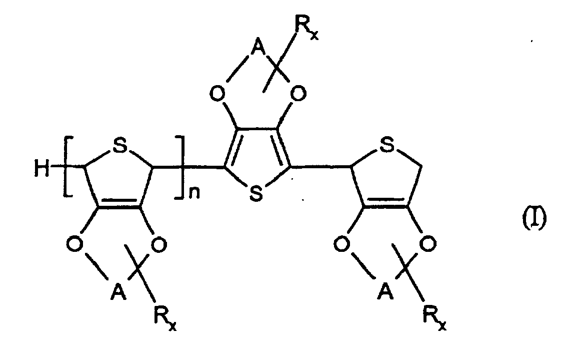 worin
A, R, x und n die in wenigstens einem der Ansprüche 1 bis 3 genannte Bedeutung haben,
**dadurch gekennzeichnet, dass** Verbindungen der allgemeinen Formel II, 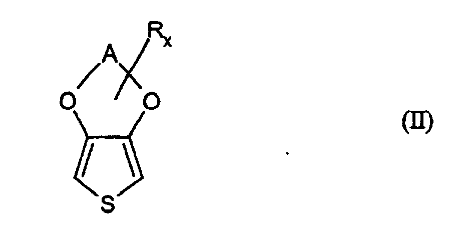 worin
A, R und x die in wenigstens einem der Ansprüche 1 bis 3 genannte Bedeutung haben,
in Gegenwart von Lewis-Säuren und/oder Protonensäuren als Katalysator miteinander umgesetzt werden.

6. Verfahren zur Herstellung von Verbindungen der allgemeinen Formel I gemäß Anspruch 5, **dadurch gekennzeichnet, dass** als Katalysator nichtoxidierende Lewis-Säuren eingesetzt werden.

7. Verfahren zur Herstellung von Verbindungen der allgemeinen Formel I gemäß Anspruch 6, **dadurch gekennzeichnet, dass** als Katalysator Lewis-Säuren ausgewählt aus der Gruppe der Bor- oder Aluminiwntrihalogenide, Phosphortrihalogenide, Titan- oder Zirkoniumtetrahalogenide, Zinn(IV)halogenide, Arsen- und Antimonhalogenide, Tantalpentahalogenide oder Zinkhalogenide eingesetzt werden.

8. Verfahren zur Herstellung von Verbindungen der allgemeinen Formel I gemäß Anspruch 7, **dadurch gekennzeichnet, dass** als Katalysator Lewis-Säuren ausgewählt aus der Gruppe Bortrifluorid, Antimonpentachlorid, Titantetrachlorid oder Zinntetrachlorid eingesetzt werden.

9. Verfahren zur Herstellung von Verbindungen der allgemeinen Formel I gemäß Anspruch 5, **dadurch gekennzeichnet, dass** als Katalysator Protonensäuren ausgewählt aus der Gruppe der Sulfon- oder Carbonsäuren oder Supersäuren eingesetzt werden.

10. Verfahren zur Herstellung von Verbindungen der allgemeinen Formel I gemäß Anspruch 9, **dadurch gekennzeichnet, dass** als Katalysator p-Toluolsulfonsäure, Methansulfonsäure, Campher-10-sulfonsäure oder Trifluoressigsäure eingesetzt wird.

11. Verwendung der Verbindungen der allgemeinen Formel I zur Herstellung von neutralen oder kationischen Polythiophenen.

12. Verfahren zur Herstellung von neutralen oder kationischen Polythiophenen der allgemeinen Formel III, 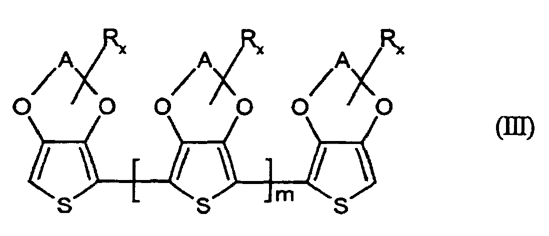 worin
A, R und x die in wenigstens einem der Ansprüche 1 bis 3 genannte Bedeutung haben und
m für eine ganze Zahl von 2 bis 200 steht,
**dadurch gekennzeichnet, dass** Verbindungen der allgemeinen Formel I gemäß wenigstens einem der Ansprüche 1 bis 3 chemisch oder elektrochemisch oxidativ polymerisiert werden.

13. Verwendung der Verbindungen der allgemeinen Formel I gemäß wenigstens einem der Ansprüche 1 bis 4 zur Herstellung von Bestandteilen elektrischer oder elektronischer Bauteile.

14. Verwendung der Verbindungen der allgemeinen Formel III, welche nach einem Verfahren gemäß Anspruch 12 hergestellt wurden, als Bestandteil von elektrischen und elektronischen Bauteilen.

15. Verwendung der Verbindungen der allgemeinen Formel III gemäß Anspruch 14 als Kathoden in Kondensatoren.

## Claims

1. Compounds having the general formula I, **characterised in that** 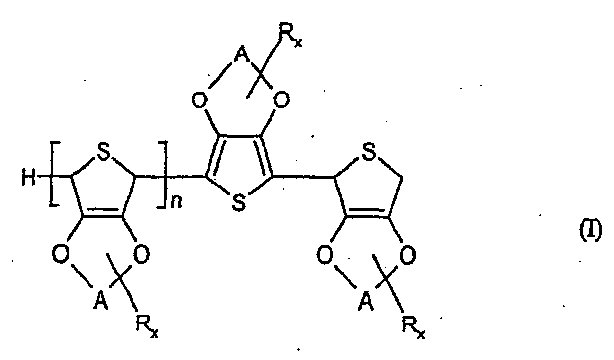
A stands for an optionally substituted C₁-C₄ alkylene radical,
R stands for one or more, identical or different linear or branched, optionally substituted C₁-C₁₈ alkyl radical(s), optionally substituted C₅-C₁₂ cycloalkyl radical(s), optionally substituted C₆-C₁₄ aryl radical(s), optionally substituted C₁-C₄ hydroxyalkyl radical(s) or one or more hydroxyl radical(s),
x stands for a whole number from 0 to 8 and
n stands for 0 or 1.

2. Compounds according to claim 1, **characterised in that**
A stands for an optionally substituted C₂ or C₃ alkylene radical,
R stands for one or more, identical or different linear or branched, optionally substituted C₁-C₁₈ alkyl radical(s), optionally substituted C₅-C₁₂ cycloalkyl radical(s), optionally substituted C₆-C₁₄ aryl radical(s), optionally substituted C₁-C₄ hydroxyalkyl radical(s) or hydroxyl radical,
x stands for a whole number from 0 to 6 and
n stands for 0 or 1.

3. Compounds according to at least one of claims 1 or 2, **characterised in that**
A stands for an optionally substituted C₂ or C₃ alkylene radical,
R stands for a linear or branched, optionally substituted C₁-C₁₄ alkyl radical, an optionally substituted C₁-C₂ hydroxyalkyl radical or a hydroxyl radical,
x stands for 0 or 1 and
n stands for 0 or 1.

4. Compounds according to at least one of claims 1 to 3, **characterised in that** they have a structure having the general formulae I-a-1 or I-b-1 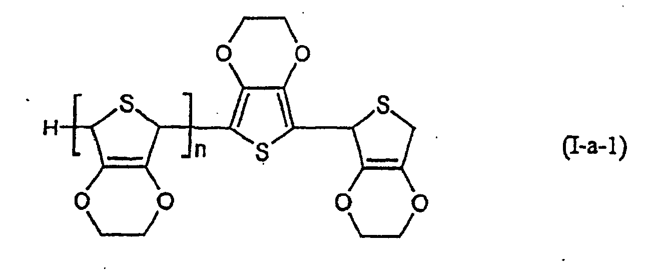 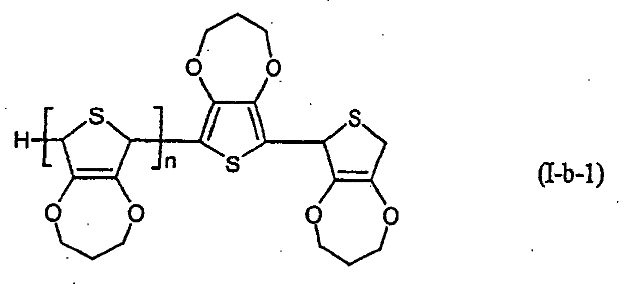 wherein
n stands for 0 or 1.

5. Process for preparing compounds having the general formula I, 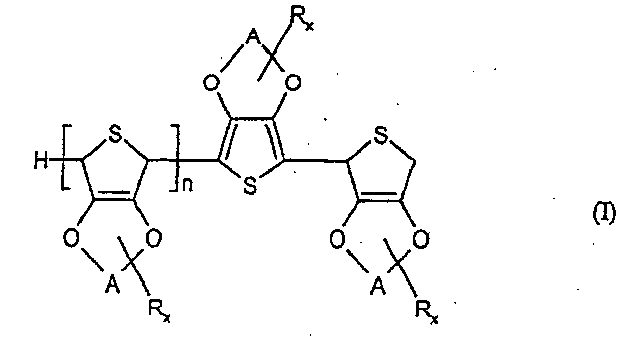 wherein
A, R, x and n have the meaning cited in at least one of claims 1 to 3,
**characterised in that** the compounds having the general formula II, 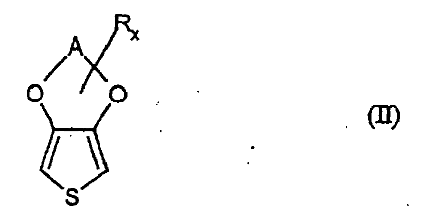 wherein
A, R and x have the meaning cited in at least one of claims 1 to 3,
are reacted with one another in the presence of Lewis acids and/or protonic acids as catalyst.

6. Process for preparing compounds having the general formula I according to claim 5, **characterised in that** non-oxidising Lewis acids are used as catalyst.

7. Process for preparing compounds having the general formula I according to claim 6, **characterised in that** Lewis acids selected from the group comprising boron or aluminium trihalides, phosphorus trihalides, titanium or zirconium tetrahalides, tin(IV) halides, arsenic and antimony halides, tantalum pentahalides or zinc halides are used as catalyst.

8. Process for preparing compounds having the general formula I according to claim 7, **characterised in that** Lewis acids selected from the group comprising boron trifluoride, antimony pentachloride, titanium tetrachloride or tin tetrachloride are used as catalyst.

9. Process for preparing compounds having the general formula I according to claim 5, **characterised in that** protonic acids selected from the group comprising sulfonic or carboxylic acids or superacids are used as catalyst.

10. Process for preparing compounds having the general formula I according to claim 9, **characterised in that** p-toluene sulfonic acid, methane sulfonic acid, camphor-10-sulfonic acid or trifluoroacetic acid are used as catalyst.

11. Use of compounds having the general formula I to prepare neutral or cationic polythiophenes.

12. Process for preparing neutral or cationic polythiophenes having the general formula III, 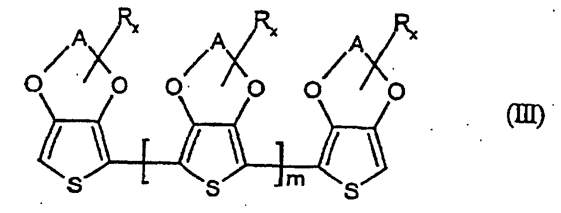 wherein
A, R and x have the meaning cited in at least one of claims 1 to 3 and
m stands for a whole number from 2 to 200,
**characterised in that** compounds having the general formula I according to at least one of claims 1 to 3 undergo chemical or electrochemical oxidative polymerisation.

13. Use of compounds having the general formula I according to at least one of claims 1 to 4 to prepare constituents of electrical or electronic components.

14. Use of compounds having the general formula III, which were prepared by a process according to claim 12, as a constituent of electrical and electronic components.

15. Use of compounds having the general formula III according to claim 14 as cathodes in capacitors.

## Revendications

1. Composés de la formule générale I, **caractérisésen ce que** : 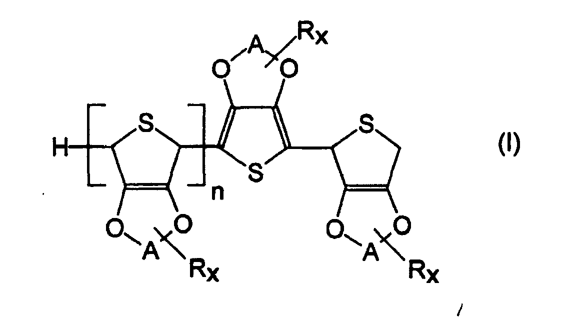
A représente un reste alcoylène en C₁-C₄ le cas échéant substitué,
R représente un ou plusieurs restes alcoyle en C₁-C₁₈ linéaires ou ramifiés, le cas échéant substitués, restes cycloalcoyle en C₅-C₁₂, le cas échéant substitués, restes aryle en C₆-C₁₄, le cas échéant substitués, restes hydroxyalcoyle en C₁-C₄, le cas échéant substitués, identiques ou différents, ou un ou plusieurs restes hydroxyle,
x représente un nombre entier allant de 0 à 8, et
n représente 0 ou 1.

2. Composés suivant la revendication 1, **caractérisés en ce que**
A représente un reste alcoylène en C₂ ou C₃ le cas échéant substitué,
R représente un ou plusieurs restes alcoyle en C₁-C₁₈ linéaires ou ramifiés, le cas échéant substitués, restes cycloalcoyle en C₅-C₁₂, le cas échéant substitués, restes aryle en C₆-C₁₄, le cas échéant substitués, restes hydroxyalcoyle en C₁-C₄, le cas échéant substitués, identiques ou différents, ou un reste hydroxyle,
x représente un nombre entier allant de 0 à 6, et
n représente 0 ou 1.

3. Composés suivant au moins une des revendications 1 ou 2, **caractérisés en ce que**
A représente un reste alcoylène en C₂ ou C₃ le cas échéant substitué,
R représente un reste alcoyle en C₁-C₁₄ linéaire ou ramifié, le cas échéant substitué, un reste hydroxyalcoyle en C₁-C₂, le cas échéant substitué, ou un reste hydroxyle,
x représente 0 ou 1, et
n représente 0 ou 1.

4. Composés suivant au moins une des revendications 1 à 3, **caractérisés en ce qu'**ils possèdent une structure de la formule générale I-a-1 ou I-b-1 : 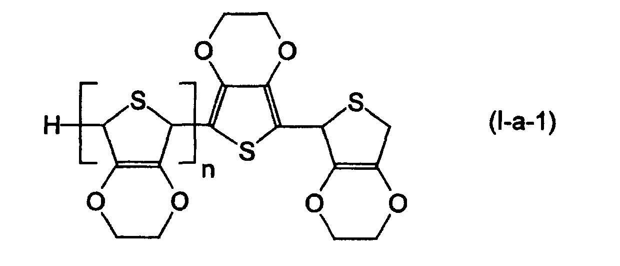 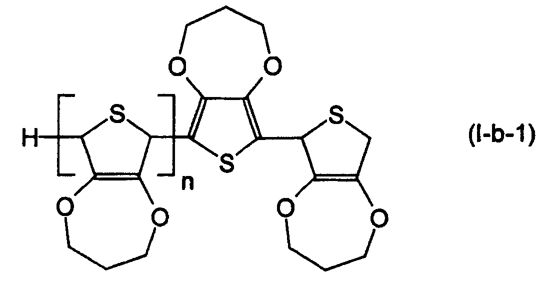 où
n représente 0 ou 1.

5. Procédé de préparation des composés de la formule générale (I) : 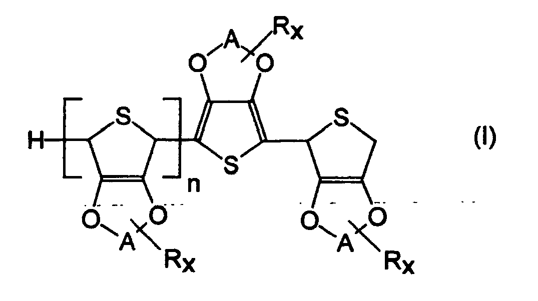 où A, R, x et n ont la signification indiquée dans au moins une des revendications 1 à 3,
**caractérisé en ce que** les composés de la formule générale (II) : 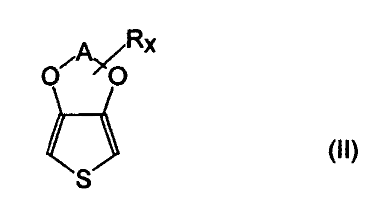 où A, R et x ont la signification indiquée dans au moins une des revendications 1 à 3,
sont mis à réagir les uns avec les autres en présence d'acides de Lewis et/ou d'acide protoniques comme catalyseur.

6. Procédé de préparation des composés de la formule générale (I) suivant la revendication 5, **caractérisé en ce que** l'on met en oeuvre comme catalyseur, des acides de Lewis non oxydants.

7. Procédé de préparation des composés de la formule générale (I) suivant la revendication 6, **caractérisé en ce que** l'on met en oeuvre comme catalyseur, des acides de Lewis choisis parmi le groupe constitué d'un trihalogénure de bore ou d'aluminium, un trihalogénure de phosphore, un tétrahalogénure de titane ou de zirconium, un halogénure d'étain (IV), un halogénure d'arsenic ou d'antimoine, un pentahalogénure de tantale ou un halogénure de zinc.

8. Procédé de préparation des composés de la formule générale (I) suivant la revendication 7, **caractérisé en ce que** l'on met en oeuvre comme catalyseur, des acides de Lewis choisis parmi le groupe du trifluorure de bore, du pentachlorure d'antimoine, du tétrachlorure de titane ou du tétrachlorure d'étain.

9. Procédé de préparation des composés de la formule générale (I) suivant la revendication 5, **caractérisé en ce que** l'on met en oeuvre comme catalyseur, des acides protoniques choisis parmi le groupe des acides sulfoniques ou carboxyliques ou des superacides.

10. Procédé de préparation des composés de la formule générale (I) suivant la revendication 9, **caractérisé en ce que** l'on met en oeuvre comme catalyseur, l'acide p-toluènesulfonique, l'acide méthanesulfonique, l'acide camphor-10-sulfonique ou l'acide trifluoroacétique.

11. Utilisation des composés de la formule générale I, pour la préparation de polythiophènes neutres ou cationiques.

12. Procédé de préparation de polythiophènes neutres ou cationiques, de la formule générale III : 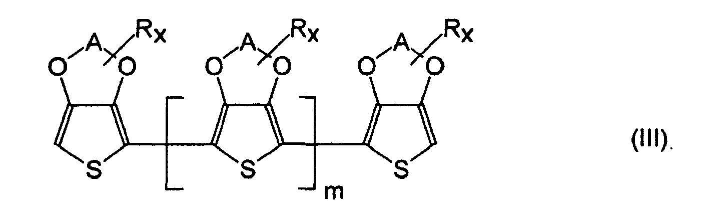 où A, R et x ont la signification indiquée dans au moins une des revendications 1 à 3, et
m représente un nombre entier allant de 2 à 200,
**caractérisé en ce que** les composés de la formule générale (I) suivant au moins une des revendications 1 à 3, sont polymérisés de manière chimique ou oxydative électrochimique.

13. Utilisation des composés de la formule générale (I) suivant au moins une des revendications 1 à 4, pour la préparation de constituants de pièces éléctriques ou électroniques.

14. Utilisation des composés de la formule générale (III), qui sont préparés selon un procédé suivant la revendication 12, comme constituant de pièces éléctriques ou électroniques.

15. Utilisation des composés de la formule générale (III), suivant la revendication 14, comme cathode dans des condensateurs.
